# EUROPEAN PATENT SPECIFICATION

(11) **EP 2 211 967 B1**
(45) Date of publication and mention of the grant of the patent: **29.07.2015**
(21) Application number: 08845941.7
(22) Date of filing: 27.10.2008
(51) Int. Cl.: A61M 25/09

(54) **ELONGATE MEDICAL DEVICE WITH A SHAPEABLE TIP**
LÄNGLICHE MEDIZINISCHE VORRICHTUNG MIT FORMBARER SPITZE
DISPOSITIF MÉDICAL ALLONGÉ AVEC UNE POINTE FAÇONNABLE

(30) Priority: 02.11.2007 US 934647
(43) Date of publication of application: 04.08.2010
(73) Proprietor: Boston Scientific Limited, Hamilton HM11 (BM)
(72) Inventor: CZYSCON, Joseph S., Plymouth Minnesota 55446 (US); REYNOLDS, Brian R., Ramsey Minnesota 55303 (US)
(74) Representative: Vossius & Partner Patentanwälte Rechtsanwälte mbB
(86) International application number: PCT/US2008/081261
(87) International publication number: WO 2009/058705

(56) References cited:
- WO-A2-2004/012804
- US-A1- 2001 007 922
- US-A1- 2005 054 950
- US-A1- 2006 293 612
- Special Materials Corp Inconel Alloy 625

## Description

The present invention pertains to medical devices. More particularly, the present invention pertains to elongated intracorporeal medical devices including a tubular member connected with other structures.

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. US 2001/0007922 A1 discloses a medical appliance having an elongated tubular shaft with a lumen extending therethrough and slots in the distal area for pressure medium entry into the lumen. A stiffening means in the form of an independent wire removably extends through the lumen with its proximal area extending proximally of the shaft. The proximal area of the shaft is affixed to a luer-lock comprising a housing with an inner cavity and a distal end forming a muff fixedly and tightly sealed on the proximal end of the shaft. Of the known medical devices and methods, each has certain advantages and disadvantages. There is an ongoing need to provide alternative medical devices as well as alternative methods for manufacturing and using medical devices.

The invention provides a medical device according to claim 1.

The above summary of some embodiments is not intended to describe each disclosed embodiment or every implementation of the present invention. The Figures, and Detailed Description, which follow, more particularly exemplify these embodiments.

The invention may be more completely understood in consideration of the following detailed description of various embodiments of the invention in connection with the accompanying drawings, in which:
Figure 1 is a plan view of an example medical device disposed in a blood vessel;
Figure 2 is a partial cross-sectional view of an example medical device;
Figure 3 is a graph depicting the flexural rigidity of an example medical device relative to position;
Figure 4 is another graph depicting the flexural rigidity of an example medical device relative to position; and
Figure 5 is another graph depicting the relative contribution of an example tubular member to the flexural rigidity of an example medical device.

While the invention is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the invention.

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

Weight percent, percent by weight, wt%, wt-%, % by weight, and the like are synonyms that refer to the concentration of a substance as the weight of that substance divided by the weight of the composition and multiplied by 100.

The following detailed description should be read with reference to the drawings in which similar elements in different drawings are numbered the same. The drawings, which are not necessarily to scale, depict illustrative embodiments and are not intended to limit the scope of the invention.

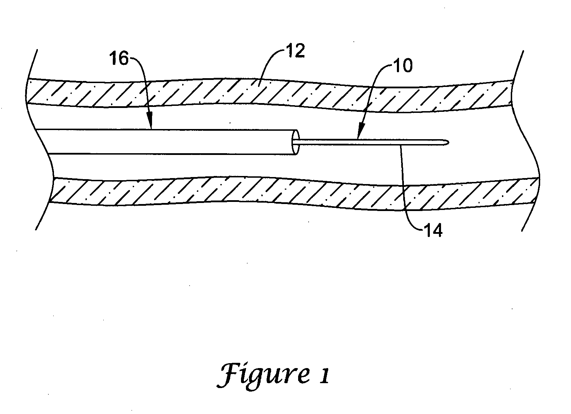

Figure 1 is a plan view of an example medical device 10, for example a guidewire, disposed in a blood vessel 12. Guidewire 10 may include a distal section 14 that may be generally configured for probing within the anatomy of a patient. Guidewire 10 may be used for intravascular procedures. For example, guidewire 10 may be used in conjunction with another medical device 16, which may take the form of a catheter, to treat and/or diagnose a medical condition. Of course, numerous other uses are known amongst clinicians for guidewires, catheters, and other similarly configured medical devices.

Although medical device 10 is depicted in several of the drawings as a guidewire, it is not intended to be limited to just being a guidewire. Indeed, medical device 10 may take the form of any suitable guiding, diagnosing, or treating device (including catheters, endoscopic instruments, laparoscopic instruments, etc., and the like) and it may be suitable for use at essentially any location and/or body lumen within a patient. For example, medical device/guidewire 10 may be suitable for use in neurological interventions, coronary interventions, peripheral interventions, etc. As such, guidewire 10 may be appropriately sized for any given intervention. For example, guidewire 10 may have an outside diameter of about 25.4 µm to 12.7 mm (0.001 to 0.5 inches) or about 38.1 µm to 1.27 mm (0015 to 0.05 inches) for neurological interventions; an outside diameter of about 25.4 µm to 12.7 mm (0.001 to 0.5 inches) or about 254 µm to 1.27 mm (0.01 to 0.05 inches) for coronary interventions; or an outside diameter of about 25.4 µm to 12.7 mm (0.01 to 0.5 inches) or about 508 µm to 1.27 mm (0.02 to 0.05 inches) for peripheral interventions. These dimensions, of course, may vary depending on, for example, the type of device (e.g., catheter, guidewire, etc.), the anatomy of the patient, and/or the goal of the intervention. In at least some embodiments, for example, guidewire 10 may be a crossing guidewire that can be used to help a clinician cross an occlusion or stenosis in vessel 12.

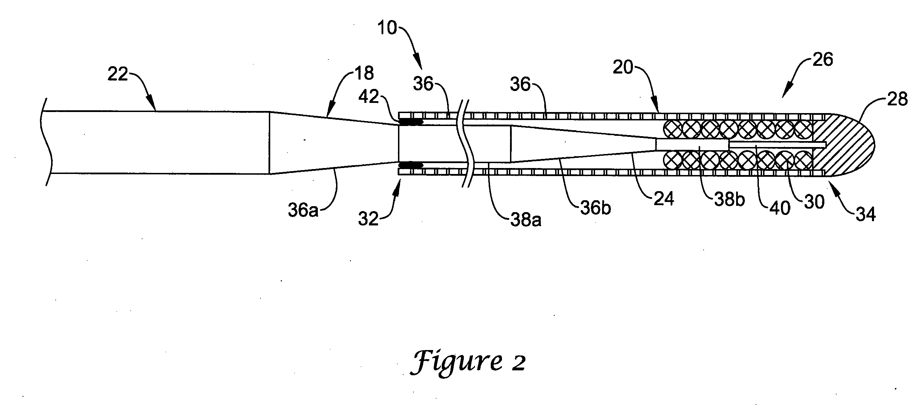

Figure 2 is a partial cross-sectional view of guidewire 10. Here it can be seen that guidewire 10 may include a core member or core wire 18 and a tubular member 20 disposed over at least a portion of core wire 18. Core wire 18 may have a proximal section 22 and a distal section 24. Distal section 24 may include one or more tapers, tapered regions, steps, stepped regions, combinations thereof, or the like. At least some of these are described in more detail below. In at least some embodiments, tubular member 20 is disposed over distal section 24 of core wire 18. Tubular member 20 and distal section 24, collectively, may define a distal tip 26 for guidewire 10. Distal tip 26 may further include a tip member 28, for example a solder ball distal tip, and a coil 30.

Distal tip 26 may be generally configured to be shapeable - i.e., distal tip 26 may be configured to be plastically deformed into a desired shape for a particular intervention. A number of structural features may contribute to the shapeability of distal tip 26. For example, the materials selected for core wire 18 and/or tubular member 20 may contribute to the overall shapeability of distal tip 26. Additionally, the relative size and/or cross-sectional area of core wire 18 and/or tubular member 20 may also contribute to shapeability.

In at least some embodiments, distal tip 26 has a flexural rigidity (e.g., based on the materials selected, the size and/or shape of the components, or any one of a number of different factors). The flexural rigidity is understood to be the amount of force required to bend distal tip 26 into a curve, arc, bend, or the like. For example, the flexural rigidity of distal tip 26 may be such that distal tip 26 will plastically deform when exposed to a force sufficient to cause about 2% or more strain on distal tip 26.

The relative contribution of the core wire 18 and the tubular member 20 to the flexural rigidity of distal tip 26 may vary relative to the longitudinal position along distal tip 26. For example, adjacent to a proximal end 32 of distal tip 26, tubular member 20 may account for about 30% or less of the flexural rigidity of distal tip 26 and adjacent to a distal end 34 of distal tip 26, tubular member 20 may account for about 70% or more of the flexural rigidity of distal tip 26. In other embodiments, adjacent to the proximal end 32 tubular member 20 may account for about 25% or less of the flexural rigidity of distal tip 26 and adjacent to the distal end 34 of distal tip 26 tubular member 20 may account for about 75% or more of the flexural rigidity of distal tip 26. In still other embodiments, adjacent to the proximal end 32 tubular member 20 may account for about 20% or less of the flexural rigidity of distal tip 26 and adjacent to the distal end 34 of distal tip 26 tubular member 20 may account for about 80% or more of the flexural rigidity of distal tip 26. In still other embodiments, adjacent to the proximal end 32 tubular member 20 may account for about 15% or less of the flexural rigidity of distal tip 26 and adjacent to the distal end 34 of distal tip 26 tubular member 20 may account for about 85% or more of the flexural rigidity of distal tip 26. In still other embodiments, adjacent to the proximal end 32 tubular member 20 may account for about 10% or less of the flexural rigidity of distal tip 26 and adjacent to the distal end 34 of distal tip 26 tubular member 20 may account for about 90% or more of the flexural rigidity of distal tip 26. In still other embodiments, adjacent to the proximal end 32 tubular member 20 may account for about 5% or less of the flexural rigidity of distal tip 26 and adjacent to the distal end 34 of distal tip 26 tubular member 20 may account for about 95% or more of the flexural rigidity of distal tip 26.

Stated another way, adjacent to the proximal end 32 of distal tip 26, the overall contribution to the shapeability of distal tip 26 may be dominated by the shapeability characteristics of core wire 18. Conversely, adjacent to the distal end 34 of distal tip 26, the overall contribution to the shapeability of distal tip 26 may be dominated by the shapeability characteristics of tubular member 20. Between proximal end 32 and distal end 34, the relative contribution transitions between the characteristics of core wire 18 and tubular member 20. This design feature may be achieved in a number of different ways including, for example, the selection of desirable materials for core wire 18 and/or tubular member 20 and by altering the total cross-sectional area (e.g., tapering) of core wire 18 and/or tubular member 20. In addition, this design feature may also be influenced by slots 36, which may be formed in tubular member 20 and are described in more detail below.

In at least some embodiments, core wire 18 (or at least the portion of core wire 18 that is part of distal tip 26, for example, distal portion 24) is made from a generally super-elastic material. For example, core wire 18 may be made from a super-elastic nickel-titanium alloy. Conversely, tubular member 20 may be from a relatively linear and/or non-super-elastic material such as nickel-chromium-molybdenum alloy (e.g., UNS: N06625 such as INCONEL® 625, UNS: N06022 such as HASTELLOY® C-22®, UNS: N10276 such as HASTELLOY® C276®, other HASTELLOY® alloys, and the like), platinum, stainless steel (such as 304V, 304L, 316LV stainless steel, mild steel, etc.), cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), linear elastic and/or non-super-elastic nickel-titanium alloy, or the like. Other materials may be utilized including any of those listed below.

With this arrangement in mind, distal tip 26 may have shapeability characteristics that are more like those of core wire 18 (e.g., generally super-elastic) adjacent to proximal end 32 whereas adjacent to distal end 34, the shapeability characteristics of distal tip 26 may more closely resemble those of tubular member 20 (e.g., generally linear and/or non-super-elastic). Thus, distal tip 26 may generally become more "linear and/or non-super-elastic" along the length thereof until, eventually, distal tip 26 becomes generally shapeable.

Other embodiments are contemplated that utilize different arrangements and materials including, for example, those materials listed below. For example, core wire 18 and/or tubular member 20 may include stainless steel, one or more linear and/or non-super elastic materials, super elastic materials (e.g., super elastic nickel-titanium alloy), or combinations thereof.

Because distal tip 26 may be shapeable, guidewire 10 may be manufactured with fewer structural components than other typical guidewires. For example, guidewire 10 may be free of having a shaping structure or ribbon. Likewise, guidewire 10 may be free of coil 30. These benefits may reduce the manufacturing costs of guidewire 10. Furthermore, because linear and/or non-super-elastic materials may be utilized for tubular member 20, which may be less expensive than super-elastic materials, further cost savings can be realized during manufacturing.

The various components of guidewire 10 may include a number of features, material compositions, and dimensions as well as variations on these characteristics. Below are listed some of these characteristics for illustration purposes. As will be appreciated, any values provided for dimensions herein are provided by way of example. Dimensions other than those provided below may be used without departing from the spirit of the invention.

Core wire 18 may have a length of about 9 to about 125 inches. Distal section 24 may make up about 5 to 80 inches of that total length, the remainder being derived from proximal section 22. In addition, core wire 18 may include a number of tapers or tapered regions (e.g., tapered regions 36a/36b). Tapered regions 36a/36b may be formed by any one of a number of different techniques, for example, by centerless grinding methods, stamping methods, and the like. The centerless grinding technique may utilize an indexing system employing sensors (e.g., optical/reflective, magnetic) to avoid excessive grinding of the connection. In addition, the centerless grinding technique may utilize a CBN or diamond abrasive grinding wheel that is well shaped and dressed to avoid grabbing core wire 18 during the grinding process. In some embodiments, core wire 18 is centerless ground using a Royal Master HI-AC centerless grinder to define tapered regions 36a/36b.

A generally constant outer diameter section 38a may extend between tapered regions 36a/36b. Located distal of tapered region 36b may be another generally constant outer diameter section 38b, followed by a shoulder or narrowed region 40 that may be narrowed abruptly (as shown) or more gently using any of the appropriate technique or methods described herein or any other suitable method. Narrowed region 40 may be coupled to tip member 28.

Core wire 18 can have a solid cross-section, but in some embodiments, can have a hollow cross-section. In yet other embodiments, core wire 18 can include a combination of areas having solid cross-sections and hollow cross sections. Moreover, core wire 18, or portions thereof, can be made of rounded wire, flattened ribbon, or other such structures having various cross-sectional geometries. The cross-sectional geometries along the length of core wire 18 can also be constant or can vary. For example, Figure 2 depicts core wire 18 as having a round cross-sectional shape. It can be appreciated that other cross-sectional shapes or combinations of shapes may be utilized without departing from the spirit of the invention. For example, the cross-sectional shape of core wire 18 may be oval, rectangular, square, polygonal, and the like, or any suitable shape.

Tubular member 20 may be bonded using any suitable technique to core wire 18. For example, tubular member 20 may be bonded to core wire 18 and coil 32 at solder or laser bond 42 (which may alternatively be any of the other bonds described herein including a weld, braze, mechanical bond, adhesive bond, or the like, or any other suitable type of bond). Indeed, portions or all of core wire 18 may include a solder coating that facilitates the joining of core wire 18 to other structures such as tubular member 20.

As indicated above, in at least some embodiments, tubular member 20 includes a plurality of cuts, apertures, and/or slots 36 formed therein. Slots 36 can be formed by methods such as micro-machining, saw-cutting (e.g., using a diamond grit embedded semiconductor dicing blade), laser cutting, electron discharge machining, grinding, milling, casting, molding, chemically etching or treating, or other known methods, and the like. In some such embodiments, the structure of the tubular member 20 is formed by cutting and/or removing portions of the tube to form slots 36. Some example embodiments of appropriate micromachining methods and other cutting methods, and structures for tubular members including slots and medical devices including tubular members are disclosed in U.S. Pat. Publication Nos. US 2003/0069522 and US 2004/0181174-A2; and U.S. Pat. Nos. 6,766,720; and 6,579,246. Some example embodiments of etching processes are described in U.S. Pat. No. 5,106,455 . It should be noted that the methods for manufacturing guidewire 10 may include forming slots 36 in tubular member 20 using any of these or other manufacturing steps.

Various embodiments of arrangements and configurations of slots 36 are contemplated. In some embodiments, at least some, if not all of slots 36 are disposed at the same or a similar angle with respect to the longitudinal axis of the tubular member 20. As shown, slots 36 can be disposed at an angle that is perpendicular, or substantially perpendicular, and/or can be characterized as being disposed in a plane that is normal to the longitudinal axis of tubular member 20. However, in other embodiments, slots 36 can be disposed at an angle that is not perpendicular, and/or can be characterized as being disposed in a plane that is not normal to the longitudinal axis of tubular member 20. Additionally, a group of one or more slots 36 may be disposed at different angles relative to another group of one or more slots 36. The distribution and/or configuration of slots 36 can also include, to the extent applicable, any of those disclosed in U.S. Pat. Publication No. US 2004/0181174.

Slots 36 may be provided to enhance the flexibility of tubular member 20 while still allowing for suitable torque transmission characteristics. Slots 36 may be formed such that one or more rings and/or turns interconnected by one or more segments and/or beams are formed in tubular member 20, and such rings and beams may include portions of tubular member 20 that remain after slots 36 are formed in the body of tubular member 20. Such an interconnected ring structure may act to maintain a relatively high degree of tortional stiffness, while maintaining a desired level of lateral flexibility. In some embodiments, some adjacent slots 36 can be formed such that they include portions that overlap with each other about the circumference of tubular member 20. In other embodiments, some adjacent slots 36 can be disposed such that they do not necessarily overlap with each other, but are disposed in a pattern that provides the desired degree of lateral flexibility.

Additionally, slots 36 can be arranged along the length of, or about the circumference of, tubular member 20 to achieve desired properties. For example, adjacent slots 36, or groups of slots 36, can be arranged in a symmetrical pattern, such as being disposed essentially equally on opposite sides about the circumference of tubular member 20, or can be rotated by an angle relative to each other about the axis of tubular member 20. Additionally, adjacent slots 36, or groups of slots 36, may be equally spaced along the length of tubular member 20, or can be arranged in an increasing or decreasing density pattern, or can be arranged in a non-symmetric or irregular pattern. Other characteristics, such as slot size, slot shape and/or slot angle with respect to the longitudinal axis of tubular member 20, can also be varied along the length of tubular member 20 in order to vary the flexibility or other properties. In other embodiments, moreover, it is contemplated that the portions of the tubular member, such as a proximal section 26, or a distal section 28, or the entire tubular member 20, may not include any such slots 36.

As suggested above, slots 36 may be formed in groups of two, three, four, five, or more slots 36, which may be located at substantially the same location along the axis of tubular member 20. Within the groups of slots 36, there may be included slots 36 that are equal in size (i.e., span the same circumferential distance around tubular member 20). In some of these as well as other embodiments, at least some slots 36 in a group are unequal in size (i.e., span a different circumferential distance around tubular member 20). Longitudinally adjacent groups of slots 36 may have the same or different configurations. For example, some embodiments of tubular member 20 include slots 36 that are equal in size in a first group and then unequally sized in an adjacent group. It can be appreciated that in groups that have two slots 36 that are equal in size, the beams (i.e., the portion of tubular member 20 remaining after slots 36 are formed therein) are aligned with the center of tubular member 20. Conversely, in groups that have two slots 36 that are unequal in size, the beams are offset from the center of tubular member 20. Some embodiments of tubular member 20 include only slots 36 that are aligned with the center of tubular member 20, only 42 that are offset from the center of tubular member 20, or slots 36 that are aligned with the center of tubular member 20 in a first group and offset from the center of tubular member 20 in another group. The amount of offset may vary depending on the depth (or length) of slots 36 and can include essentially any suitable distance.

The materials that can be used for the various components of guidewire 10 may include those commonly associated with medical devices. For example, core wire 18, and/or tubular member 20, and the like may be made from a metal, metal alloy, polymer (some examples of which are disclosed below), a metal-polymer composite, combinations thereof, and the like, or any other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; combinations thereof; and the like; or any other suitable material.

As alluded to above, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2-0.44% strain before plastically deforming.

In some embodiments, the linear elastic and/or non-super-elastic nickel-titanium alloy is an alloy that does not show any martensite/austenite phase changes that are detectable by DSC and DMTA analysis over a large temperature range. For example, in some embodiments, there may be no martensite/austenite phase changes detectable by DSC and DMTA analysis in the range of about -60°C to about 120°C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties and has essentially no yield point.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803 . Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of core wire 18 and/or tubular member 20 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of device 10 in determining its location. Some examples of radiopaque materials can include, but are not limited to, gold, platinum, palladium, tantalum, tungsten alloy, polymer material loaded with a radiopaque filler, and the like. Additionally, radiopaque marker bands and/or coils may be incorporated into the design of guidewire 10 to achieve the same result.

In some embodiments, a degree of MRI compatibility is imparted into guidewire 10. For example, to enhance compatibility with Magnetic Resonance Imaging (MRI) machines, it may be desirable to make core wire 18 and/or tubular member 20, or other portions of the guidewire 10, in a manner that would impart a degree of MRI compatibility. For example, core wire 18 and/or tubular member 20, or portions thereof, may be made of a material that does not substantially distort the image and create substantial artifacts (artifacts are gaps in the image). Certain ferromagnetic materials, for example, may not be suitable because they may create artifacts in an MRI image. Core wire 18 and/or tubular member 20, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

Referring now to core wire 18, the entire core wire 18 can be made of the same material along its length, or in some embodiments, can include portions or sections made of different materials that are joined, coupled, or otherwise connected together using a suitable connector. Essentially any suitable configuration and/or structure can be utilized for a connector including those connectors described in U.S. Patent Nos. 6,918,882 and 7,071,197 and/or in U.S. Patent Pub. No. US 2006-0122537 . For example, the different portions of core wire 18 can be connected using welding (including laser welding), soldering, brazing, adhesive, or the like, or combinations thereof. These techniques can be utilized regardless of whether or not a connector is utilized.

In some embodiments, proximal section 22 and distal section 24 of core wire 18 may be formed of different materials, for example materials having different moduli of elasticity, resulting in a difference in flexibility. In some embodiments, the material used to construct proximal section 22 can be relatively stiff for pushability and torqueability, and the material used to construct distal section 24 can be relatively flexible by comparison for better lateral trackability and steerability. For example, proximal section 22 can be formed of straightened 304v stainless steel wire or ribbon (and/or linear and/or non-super-elastic nickel-titanium alloy) and distal section 24 can be formed of a straightened super elastic or linear elastic alloy, for example a nickel-titanium alloy wire or ribbon.

A sheath or covering (not shown) may be disposed over portions or all of core wire 18 and/or tubular member 20 that may define a generally smooth outer surface for guidewire 10. In other embodiments, however, such a sheath or covering may be absent from a portion of all of guidewire 10, such that tubular member 20 and/or core wire 18 may form the outer surface. The sheath may be made from a polymer or any other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane, polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, polyolefin, polystyrene, epoxy, polyvinylidene chloride (PVdC), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6% LCP.

In some embodiments, the exterior surface of the guidewire 10 (including, for example, the exterior surface of core wire 18 and/or the exterior surface of tubular member 20) may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over portion of core wire 18 and/or tubular member, or other portions of device 10. Alternatively, the sheath may comprise a lubricious, hydrophilic, protective, or other type of coating. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The same may be true of tip member 30. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present invention.

### Examples

These examples are merely for illustrative purposes only and are not meant to be limiting on the scope of the appended claims.

### Example 1

An example guidewire was constructed. This guidewire may be similar to guidewire The guidewire included a core wire having a distal section made from linear and/or non-super-elastic nickel titanium alloy. A tubular member having a plurality of slots formed therein was disposed over the distal section of the core wire. The tubular member was made of a nickel-chromium-molybdenum alloys (e.g., UNS: N06625 such as INCONEL® 625). A platinum coil was disposed within the tubular member adjacent to the core wire. The flexural rigidity (EI; where E is the modulus of elasticity and I is the second moment of inertia) was calculated. The data is presented in Table 1.

**Table 1. Flexural Rigidity in an Example Guidewire.**

| **Position** | **Tubular member EI** | **Coil EI** | **Core wire Diameter** | **Core Wire EI** | **Core Wire EI** | **Total EI** |
|---|---|---|---|---|---|---|
| [in.] | [lb-in^2] | [lb-in^2] | [in.] | [lb-in^2] | [lb-in^2] | [lb-in^2] |
| | | | | | | |
| 0.00 | 1.65E-05 | 1.75E-06 | | 6.72E-06 | | 2.50E-05 |
| 0.05 | 1.65E-05 | 1.75E-06 | | 6.72E-06 | | 2.50E-05 |
| 0.10 | 1.65E-05 | 1.75E-06 | | 6.72E-06 | | 2.50E-05 |
| 0.15 | 1.65E-05 | 1.75E-06 | | 6.72E-06 | | 2.50E-05 |
| 0.20 | 1.65E-05 | 1.75E-06 | | 6.72E-06 | | 2.50E-05 |
| 0.25 | 1.65E-05 | 1.75E-06 | | 6.72E-06 | | 2.50E-05 |
| 0.30 | 1.65E-05 | 1.75E-06 | | 6.72E-06 | | 2.50E-05 |
| 0.35 | 1.65E-05 | 1.75E-06 | | 6.72E-06 | | 2.50E-05 |
| 0.40 | 1.65E-05 | 1.75E-06 | | 6.72E-06 | | 2.50E-05 |
| 0.41 | 1.65E-05 | 1.75E-06 | | 6.72E-06 | | 2.50E-05 |
| 0.45 | 1.65E-05 | 1.75E-06 | | 6.72E-06 | | 2.50E-05 |
| 0.50 | 1.65E-05 | 1.75E-06 | | 6.72E-06 | | 2.50E-05 |
| 0.55 | 0.0000166 | 1.75E-06 | | 6.72E-06 | | 2.51E-05 |
| 0.60 | 0.0000168 | 1.75E-06 | | 6.72E-06 | | 2.53E-05 |
| 0.65 | 0.0000170 | 1.75E-06 | 2.80E-03 | | 2.41E-05 | 4.29E-05 |
| 0.70 | 0.0000173 | 1.75E-06 | 2.80E-03 | | 2.41E-05 | 4.31E-05 |
| 0.75 | 0.0000176 | 1.75E-06 | 2.80E-03 | | 2.41E-05 | 4.35E-05 |
| 0.80 | 0.0000180 | 1.75E-06 | 2.80E-03 | | 2.41E-05 | 4.39E-05 |
| 0.81 | 0.0000181 | 1.75E-06 | 2.83E-03 | | 2.51E-05 | 4.50E-05 |
| 0.85 | 0.0000185 | 1.75E-06 | 2.94E-03 | | 2.95E-05 | 4.97E-05 |
| 0.90 | 0.0000190 | 1.75E-06 | 3.09E-03 | | 3.57E-05 | 5.64E-05 |
| 0.95 | 0.0000197 | 1.75E-06 | 3.23E-03 | | 4.28E-05 | 6.42E-05 |
| 1.00 | 0.0000205 | 1.75E-06 | 3.37E-03 | | 5.09E-05 | 7.31E-05 |
| 1.05 | 0.0000214 | 1.75E-06 | 3.52E-03 | | 6.01E-05 | 8.33E-05 |
| 1.10 | 0.0000224 | 1.75E-06 | 3.66E-03 | | 7.05E-05 | 9.47E-05 |
| 1.15 | 0.0000237 | 1.75E-06 | 3.80E-03 | | 8.23E-05 | 1.08E-04 |
| 1.20 | 0.0000250 | 1.75E-06 | 3.95E-03 | | 9.54E-05 | 1.22E-04 |
| 1.25 | 0.0000266 | | 4.09E-03 | | 1.10E-04 | 1.37E-04 |
| 1.30 | 0.0000284 | | 4.23E-03 | | 1.26E-04 | 1.55E-04 |
| 1.35 | 0.0000303 | | 4.38E-03 | | 1.44E-04 | 1.75E-04 |
| 1.40 | 0.0000325 | | 4.52E-03 | | 1.64E-04 | 1.97E-04 |
| 1.45 | 0.0000350 | | 4.67E-03 | | 1.86E-04 | 2.21E-04 |
| 1.50 | 0.0000377 | | 4.81E-03 | | 2.10E-04 | 2.48E-04 |
| 1.55 | 0.0000407 | | 4.95E-03 | | 2.36E-04 | 2.77E-04 |
| 1.60 | 0.0000440 | | 5.10E-03 | | 2.65E-04 | 3.09E-04 |
| 1.65 | 0.0000477 | | 5.24E-03 | | 2.96E-04 | 3.44E-04 |
| 1.70 | 0.0000517 | | 5.38E-03 | | 3.30E-04 | 3.81E-04 |
| 1.75 | 0.0000560 | | 5.53E-03 | | 3.66E-04 | 4.22E-04 |
| 1.80 | 0.0000608 | | 5.67E-03 | | 4.06E-04 | 4.67E-04 |
| 1.85 | 0.0000659 | | 5.81E-03 | | 4.48E-04 | 5.14E-04 |
| 1.90 | 0.0000715 | | 5.96E-03 | | 4.94E-04 | 5.66E-04 |
| 1.95 | 0.0000776 | | 6.10E-03 | | 5.44E-04 | 6.21E-04 |
| 1.97 | 0.0000802 | | 6.16E-03 | | 5.64E-04 | 6.45E-04 |
| 2.00 | 0.0000841 | | 6.24E-03 | | 5.97E-04 | 6.81E-04 |
| 2.05 | 0.0000912 | | 6.39E-03 | | 6.53E-04 | 7.45E-04 |
| 2.10 | 0.0000988 | | 6.53E-03 | | 7.14E-04 | 8.13E-04 |
| 2.15 | 0.0001069 | | 6.67E-03 | | 7.79E-04 | 8.86E-04 |
| 2.20 | 0.0001157 | | 6.82E-03 | | 8.48E-04 | 9.64E-04 |
| 2.25 | 0.0001250 | | 6.96E-03 | | 9.22E-04 | 1.05E-03 |
| 2.30 | 0.0001350 | | 7.10E-03 | | 1.00E-03 | 1.14E-03 |
| 2.35 | 0.0001457 | | 7.25E-03 | | 1.08E-03 | 1.23E-03 |
| 2.40 | 0.0001570 | | 7.39E-03 | | 1.17E-03 | 1.33E-03 |
| 2.45 | 0.0001691 | | 7.53E-03 | | 1.27E-03 | 1.43E-03 |
| 2.50 | 0.0001820 | | 7.68E-03 | | 1.36E-03 | 1.55E-03 |
| 2.55 | 0.0001957 | | 7.82E-03 | | 1.47E-03 | 1.67E-03 |
| 2.60 | 0.0002102 | | 7.97E-03 | | 1.58E-03 | 1.79E-03 |
| 2.65 | 0.0002255 | | 8.11E-03 | | 1.70E-03 | 1.92E-03 |
| 2.70 | 0.0002418 | | 8.25E-03 | | 1.82E-03 | 2.06E-03 |
| 2.75 | 0.0002590 | | 8.40E-03 | | 1.95E-03 | 2.21E-03 |
| 2.78 | 0.0002679 | | 8.47E-03 | | 2.02E-03 | 2.29E-03 |
| 2.80 | 0.0002771 | | 8.54E-03 | | 2.09E-03 | 2.37E-03 |
| 2.85 | 0.0002962 | | 8.68E-03 | | 2.23E-03 | 2.53E-03 |
| 2.90 | 0.0003164 | | 8.83E-03 | | 2.38E-03 | 2.70E-03 |
| 2.95 | 0.0003376 | | 8.97E-03 | | 2.54E-03 | 2.88E-03 |
| 3.00 | 3.60E-04 | | 9.11E-03 | | 2.71E-03 | 3.07E-03 |
| 3.05 | 0.0003600 | | 9.26E-03 | | 2.88E-03 | 3.24E-03 |
| 3.10 | 0.0003600 | | 9.40E-03 | | 3.07E-03 | 3.43E-03 |
| 3.15 | 0.0003600 | | 9.40E-03 | | 3.07E-03 | 3.43E-03 |
| 3.20 | 0.0003600 | | 9.40E-03 | | 3.07E-03 | 3.43E-03 |
| 3.25 | 0.0003600 | | 9.40E-03 | | 3.07E-03 | 3.43E-03 |
| 3.30 | 0.0003600 | | 9.40E-03 | | 3.07E-03 | 3.43E-03 |
| 3.35 | 0.0003600 | | 9.40E-03 | | 3.07E-03 | 3.43E-03 |
| 3.40 | 0.0003600 | | 9.40E-03 | | 3.07E-03 | 3.43E-03 |
| 3.45 | 0.0003600 | | 9.40E-03 | | 3.07E-03 | 3.43E-03 |
| 3.50 | 0.0003600 | | 9.40E-03 | | 3.07E-03 | 3.43E-03 |
| 3.55 | 0.0003600 | | 9.40E-03 | | 3.07E-03 | 3.43E-03 |
| 3.60 | 0.0003600 | | 9.40E-03 | | 3.07E-03 | 3.43E-03 |
| 3.65 | 0.0003600 | | 9.40E-03 | | 3.07E-03 | 3.43E-03 |
| 3.70 | 0.0003600 | | 9.40E-03 | | 3.07E-03 | 3.43E-03 |
| 3.75 | 0.0003600 | | 9.40E-03 | | 3.07E-03 | 3.43E-03 |
| 3.80 | 0.0003600 | | 9.40E-03 | | 3.07E-03 | 3.43E-03 |
| 3.85 | 0.0003600 | | 9.40E-03 | | 3.07E-03 | 3.43E-03 |
| 3.90 | 0.0003600 | | 9.40E-03 | | 3.07E-03 | 3.43E-03 |
| 3.94 | 0.0003600 | | 9.40E-03 | | 3.07E-03 | 3.43E-03 |
| 3.95 | 0.0003600 | | 9.40E-03 | | 3.07E-03 | 3.43E-03 |
| 4.00 | 0.0003600 | | 9.40E-03 | | 3.07E-03 | 3.43E-03 |
| 4.05 | 0.0003600 | | 9.40E-03 | | 3.07E-03 | 3.43E-03 |
| 4.10 | 0.0003600 | | 9.40E-03 | | 3.07E-03 | 3.43E-03 |
| 4.15 | 0.0003600 | | 9.40E-03 | | 3.07E-03 | 3.43E-03 |
| 4.20 | 0.0003600 | | 9.40E-03 | | 3.07E-03 | 3.43E-03 |
| 4.25 | 0.0003600 | | 9.40E-03 | | 3.07E-03 | 3.43E-03 |
| 4.30 | 0.0003600 | | 9.40E-03 | | 3.07E-03 | 3.43E-03 |
| 4.35 | 0.0003600 | | 9.40E-03 | | 3.07E-03 | 3.43E-03 |
| 4.40 | 0.0003600 | | 9.40E-03 | | 3.07E-03 | 3.43E-03 |
| 4.45 | 0.0003600 | | 9.40E-03 | | 3.07E-03 | 3.43E-03 |
| 4.50 | 0.0003600 | | 9.40E-03 | | 3.07E-03 | 3.43E-03 |
| 4.55 | 0.0003600 | | 9.40E-03 | | 3.07E-03 | 3.43E-03 |
| 4.60 | 0.0003600 | | 9.40E-03 | | 3.07E-03 | 3.43E-03 |
| 4.65 | 0.0003600 | | 9.40E-03 | | 3.07E-03 | 3.43E-03 |
| 4.70 | 0.0003600 | | 9.40E-03 | | 3.07E-03 | 3.43E-03 |
| 4.75 | 0.0003600 | | 9.40E-03 | | 3.07E-03 | 3.43E-03 |
| 4.80 | 0.0003600 | | 9.40E-03 | | 3.07E-03 | 3.43E-03 |
| 4.85 | 0.0003600 | | 9.40E-03 | | 3.07E-03 | 3.43E-03 |
| 4.90 | 0.0003600 | | 9.40E-03 | | 3.07E-03 | 3.43E-03 |
| 4.95 | 0.0003600 | | 9.40E-03 | | 3.07E-03 | 3.43E-03 |
| 5.00 | 0.0003600 | | 9.40E-03 | | 3.07E-03 | 3.43E-03 |
| 5.05 | 0.0003600 | | 9.40E-03 | | 3.07E-03 | 3.43E-03 |
| 5.10 | 0.0003600 | | 9.40E-03 | | 3.07E-03 | 3.43E-03 |
| 5.15 | 0.0003600 | | 9.40E-03 | | 3.07E-03 | 3.43E-03 |
| 5.20 | 0.0003600 | | 9.40E-03 | | 3.07E-03 | 3.43E-03 |
| 5.25 | 0.0003600 | | 9.40E-03 | | 3.07E-03 | 3.43E-03 |
| 5.30 | 0.0003600 | | 9.40E-03 | | 3.07E-03 | 3.43E-03 |
| 5.35 | 0.0003600 | | 9.40E-03 | | 3.07E-03 | 3.43E-03 |
| 5.40 | 0.0003600 | | 9.40E-03 | | 3.07E-03 | 3.43E-03 |
| 5.45 | 0.0003600 | | 9.40E-03 | | 3.07E-03 | 3.43E-03 |
| 5.50 | 0.0003600 | | 9.40E-03 | | 3.07E-03 | 3.43E-03 |
| 5.55 | 0.0003600 | | 9.40E-03 | | 3.07E-03 | 3.43E-03 |
| 5.60 | 0.0003600 | | 9.40E-03 | | 3.07E-03 | 3.43E-03 |
| 5.65 | 0.0003600 | | 9.40E-03 | | 3.07E-03 | 3.43E-03 |
| 5.70 | 0.0003600 | | 9.40E-03 | | 3.07E-03 | 3.43E-03 |
| 5.75 | 0.0003600 | | 9.40E-03 | | 3.07E-03 | 3.43E-03 |
| 5.80 | 0.0003600 | | 9.40E-03 | | 3.07E-03 | 3.43E-03 |
| 5.85 | 0.0003600 | | 9.40E-03 | | 3.07E-03 | 3.43E-03 |
| 5.90 | 0.0003600 | | 9.40E-03 | | 3.07E-03 | 3.43E-03 |
| 5.95 | 0.0003600 | | 9.40E-03 | | 3.07E-03 | 3.43E-03 |
| 6.00 | 0.0003600 | | 9.40E-03 | | 3.07E-03 | 3.43E-03 |
| 6.05 | 0.0003600 | | 9.40E-03 | | 3.07E-03 | 3.43E-03 |
| 6.10 | 0.0003600 | | 9.40E-03 | | 3.07E-03 | 3.43E-03 |
| 6.15 | 0.0003600 | | 9.40E-03 | | 3.07E-03 | 3.43E-03 |
| 6.20 | 0.0003600 | | 9.40E-03 | | 3.07E-03 | 3.43E-03 |
| 6.25 | 0.0003600 | | 9.40E-03 | | 3.07E-03 | 3.43E-03 |
| 6.30 | 0.0003600 | | 9.40E-03 | | 3.07E-03 | 3.43E-03 |
| 6.35 | 0.0003600 | | 9.40E-03 | | 3.07E-03 | 3.43E-03 |
| 6.40 | 0.0003600 | | 9.40E-03 | | 3.07E-03 | 3.43E-03 |
| 6.45 | 0.0003600 | | 9.40E-03 | | 3.07E-03 | 3.43E-03 |
| 6.50 | 0.0003600 | | 9.40E-03 | | 3.07E-03 | 3.43E-03 |
| 6.55 | 0.0003600 | | 9.40E-03 | | 3.07E-03 | 3.43E-03 |
| 6.60 | 0.0003600 | | 9.40E-03 | | 3.07E-03 | 3.43E-03 |
| 6.65 | 0.0003600 | | 9.40E-03 | | 3.07E-03 | 3.43E-03 |
| 6.70 | 0.0003600 | | 9.40E-03 | | 3.07E-03 | 3.43E-03 |
| 6.75 | 0.0003600 | | 9.40E-03 | | 3.07E-03 | 3.43E-03 |
| 6.80 | 0.0003600 | | 9.40E-03 | | 3.07E-03 | 3.43E-03 |
| 6.85 | 0.0003600 | | 9.40E-03 | | 3.07E-03 | 3.43E-03 |
| 6.90 | 0.0003600 | | 9.40E-03 | | 3.07E-03 | 3.43E-03 |
| 6.95 | 0.0003600 | | 9.40E-03 | | 3.07E-03 | 3.43E-03 |
| 7.00 | 0.0003600 | | 9.40E-03 | | 3.07E-03 | 3.43E-03 |
| 7.05 | | | 9.40E-03 | | 3.07E-03 | 3.07E-03 |
| 7.10 | | | 9.40E-03 | | 3.07E-03 | 3.07E-03 |
| 7.15 | | | 9.40E-03 | | 3.07E-03 | 3.07E-03 |
| 7.20 | | | 9.40E-03 | | 3.07E-03 | 3.07E-03 |
| 7.25 | | | 9.40E-03 | | 3.07E-03 | 3.07E-03 |
| 7.30 | | | 9.57E-03 | | 3.30E-03 | 3.30E-03 |
| 7.35 | | | 9.74E-03 | | 3.54E-03 | 3.54E-03 |
| 7.40 | | | 9.91 E-03 | | 3.79E-03 | 3.79E-03 |
| 7.45 | | | 1.01E-02 | | 4.06E-03 | 4.06E-03 |
| 7.50 | | | 1.03E-02 | | 4.34E-03 | 4.34E-03 |
| 7.55 | | | 1.04E-02 | | 4.64E-03 | 4.64E-03 |
| 7.60 | | | 1.06E-02 | | 4.95E-03 | 4.95E-03 |
| 7.65 | | | 1.08E-02 | | 5.28E-03 | 5.28E-03 |
| 7.70 | | | 1.09E-02 | | 5.62E-03 | 5.62E-03 |
| 7.75 | | | 1.11E-02 | | 5.98E-03 | 5.98E-03 |
| 7.80 | | | 1.13E-02 | | 6.36E-03 | 6.36E-03 |
| 7.85 | | | 1.15E-02 | | 6.75E-03 | 6.75E-03 |
| 7.90 | | | 1.16E-02 | | 7.16E-03 | 7.16E-03 |
| 7.95 | | | 1.18E-02 | | 7.59E-03 | 7.59E-03 |
| 8.00 | | | 1.20E-02 | | 8.04E-03 | 8.04E-03 |
| 8.05 | | | 1.21E-02 | | 8.51E-03 | 8.51E-03 |
| 8.10 | | | 1.23E-02 | | 9.00E-03 | 9.00E-03 |
| 8.15 | | | 1.25E-02 | | 9.51E-03 | 9.51E-03 |
| 8.20 | | | 1.26E-02 | | 1.00E-02 | 1.00E-02 |
| 8.25 | | | 1.28E-02 | | 1.06E-02 | 1.06E-02 |
| 8.30 | | | 1.30E-02 | | 1.12E-02 | 1.12E-02 |
| 8.35 | | | 1.32E-02 | | 1.18E-02 | 1.18E-02 |
| 8.40 | | | 1.33E-02 | | 1.24E-02 | 1.24E-02 |
| 8.45 | | | 1.35E-02 | | 1.30E-02 | 1.30E-02 |
| 8.50 | | | 1.35E-02 | | 1.30E-02 | 1.30E-02 |
| 8.55 | | | 1.35E-02 | | 1.30E-02 | 1.30E-02 |
| 8.60 | | | 1.35E-02 | | 1.30E-02 | 1.30E-02 |
| 8.65 | | | 1.35E-02 | | 1.30E-02 | 1.30E-02 |
| 8.70 | | | 1.35E-02 | | 1.30E-02 | 1.30E-02 |
| 8.75 | | | 1.35E-02 | | 1.30E-02 | 1.30E-02 |
| 8.80 | | | 1.35E-02 | | 1.30E-02 | 1.30E-02 |
| 8.85 | | | 1.35E-02 | | 1.30E-02 | 1.30E-02 |
| 8.90 | | | 1.35E-02 | | 1.30E-02 | 1.30E-02 |
| 8.95 | | | 1.35E-02 | | 1.30E-02 | 1.30E-02 |
| 9.00 | | | 1.35E-02 | | 1.30E-02 | 1.30E-02 |
| 9.25 | | | 1.35E-02 | | 1.30E-02 | 1.30E-02 |
| 9.50 | | | 1.35E-02 | | 1.30E-02 | 1.30E-02 |
| 9.75 | | | 1.35E-02 | | 1.30E-02 | 1.30E-02 |
| 10.00 | | | 1.35E-02 | | 1.30E-02 | 1.30E-02 |
| 10.25 | | | 1.35E-02 | | 1.30E-02 | 1.30E-02 |
| 10.50 | | | 1.35E-02 | | 1.30E-02 | 1.30E-02 |
| 10.75 | | | 1.35E-02 | | 1.30E-02 | 1.30E-02 |
| 11.00 | | | 1.35E-02 | | 1.30E-02 | 1.30E-02 |
| 11.25 | | | 1.35E-02 | | 1.30E-02 | 1.30E-02 |
| 11.50 | | | 1.35E-02 | | 1.30E-02 | 1.30E-02 |
| 11.75 | | | 1.35E-02 | | 1.30E-02 | 1.30E-02 |
| 12.00 | | | 1.35E-02 | | 1.30E-02 | 1.30E-02 |

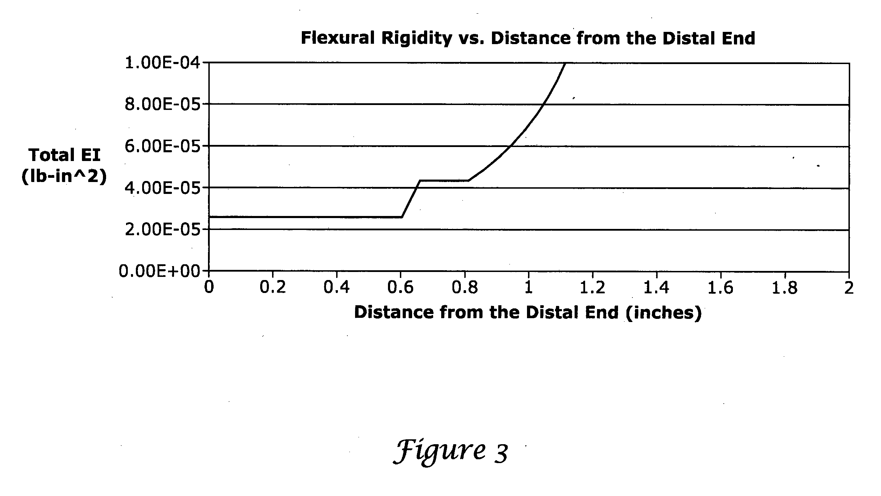

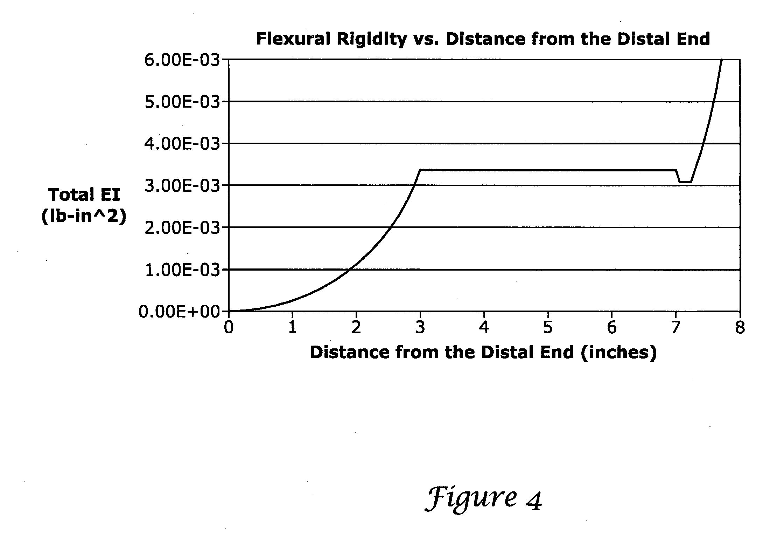

The total EI was plotted versus position (i.e., the distance from the distal end of the guidewire) in Figure 3 (distal end to about 1.1 inches from the distal end) and in Figure 4 (distal end to about 7.5 inches from the distal end).

### Example 2

The percent contribution of the tubular member to the total guidewire EL relative to the distance from the distal end of the guidewire in Example 1 was determined. The results are listed in Table 2.

**Table 2. Percent Contribution of the Tubular Member to the Total Guidewire EL Relative to the Distance from the Distal End of the Guidewire.**

| **Position** | | **% Contribution of the Tubular Member to the Total Guidewire EI** |
|---|---|---|
| [in.] | | |
| | | |
| 0.00 | | 66% |
| 0.05 | | 66% |
| 0.10 | | 66% |
| 0.15 | | 66% |
| 0.20 | | 66% |
| 0.25 | | 66% |
| 0.30 | | 66% |
| 0.35 | | 66% |
| 0.40 | | 66% |
| 0.41 | | 66% |
| 0.45 | | 66% |
| 0.50 | | 66% |
| 0.55 | | 66% |
| 0.60 | | 66% |
| 0.65 | | 40% |
| 0.70 | | 40% |
| 0.75 | | 40% |
| 0.80 | | 41% |
| 0.81 | | 40% |
| 0.85 | | 37% |
| 0.90 | | 34% |
| 0.95 | | 31% |
| 1.00 | | 28% |
| 1.05 | | 26% |
| 1.10 | | 24% |
| 1.15 | | 22% |
| 1.20 | | 20% |
| 1.25 | | 19% |
| 1.30 | | 18% |
| 1.35 | | 17% |
| 1.40 | | 17% |
| 1.45 | | 16% |
| 1.50 | | 15% |
| 1.55 | | 15% |
| 1.60 | | 14% |
| 1.65 | | 14% |
| 1.70 | | 14% |
| 1.75 | | 13% |
| 1.80 | | 13% |
| 1.85 | | 13% |
| 1.90 | | 13% |
| 1.95 | | 12% |
| 1.97 | | 12% |
| 2.00 | | 12% |
| 2.05 | | 12% |
| 2.10 | | 12% |
| 2.15 | | 12% |
| 2.20 | | 12% |
| 2.25 | | 12% |
| 2.30 | | 12% |
| 2.35 | | 12% |
| 2.40 | | 12% |
| 2.45 | | 12% |
| 2.50 | | 12% |
| 2.55 | | 12% |
| 2.60 | | 12% |
| 2.65 | | 12% |
| 2.70 | | 12% |
| 2.75 | | 12% |
| 2.78 | | 12% |
| 2.80 | | 12% |
| 2.85 | | 12% |
| 2.90 | | 12% |
| 2.95 | | 12% |
| 3.00 | | 12% |
| 3.05 | | 11% |
| 3.10 | | 11% |
| 3.15 | | 11% |
| 3.20 | | 11% |
| 3.25 | | 11% |
| 3.30 | | 11% |
| 3.35 | | 11% |
| 3.40 | | 11% |
| 3.45 | | 11% |
| 3.50 | | 11% |
| 3.55 | | 11% |
| 3.60 | | 11% |
| 3.65 | | 11% |
| 3.70 | | 11% |
| 3.75 | | 11% |
| 3.80 | | 11% |
| 3.85 | | 11% |
| 3.90 | | 11% |
| 3.94 | | 11% |
| 3.95 | | 11% |
| 4.00 | | 11% |
| 4.05 | | 11% |
| 4.10 | | 11% |
| 4.15 | | 11% |
| 4.20 | | 11% |
| 4.25 | | 11% |
| 4.30 | | 11% |
| 4.35 | | 11% |
| 4.40 | | 11% |
| 4.45 | | 11% |
| 4.50 | | 11% |
| 4.55 | | 11% |
| 4.60 | | 11% |
| 4.65 | | 11% |
| 4.70 | | 11% |
| 4.75 | | 11% |
| 4.80 | | 11% |
| 4.85 | | 11% |
| 4.90 | | 11% |
| 4.95 | | 11% |
| 5.00 | | 11% |
| 5.05 | | 11% |
| 5.10 | | 11% |
| 5.15 | | 11% |
| 5.20 | | 11% |
| 5.25 | | 11% |
| 5.30 | | 11% |
| 5.35 | | 11% |
| 5.40 | | 11% |
| 5.45 | | 11% |
| 5.50 | | 11% |
| 5.55 | | 11% |
| 5.60 | | 11% |
| 5.65 | | 11% |
| 5.70 | | 11% |
| 5.75 | | 11% |
| 5.80 | | 11% |
| 5.85 | | 11% |
| 5.90 | | 11% |
| 5.95 | | 11% |
| 6.00 | | 11% |
| 6.05 | | 11% |
| 6.10 | | 11% |
| 6.15 | | 11% |
| 6.20 | | 11% |
| 6.25 | | 11% |
| 6.30 | | 11% |
| 6.35 | | 11% |
| 6.40 | | 11% |
| 6.45 | | 11% |
| 6.50 | | 11% |
| 6.55 | | 11% |
| 6.60 | | 11% |
| 6.65 | | 11% |
| 6.70 | | 11% |
| 6.75 | | 11% |
| 6.80 | | 11% |
| 6.85 | | 11% |
| 6.90 | | 11% |
| 6.95 | | 11% |
| 7.00 | | 11% |
| 7.05 | | 0% |
| 7.10 | | 0% |
| 7.15 | | 0% |
| 7.20 | | 0% |
| 7.25 | | 0% |
| 7.30 | | 0% |
| 7.35 | | 0% |
| 7.40 | | 0% |
| 7.45 | | 0% |
| 7.50 | | 0% |
| 7.55 | | 0% |
| 7.60 | | 0% |
| 7.65 | | 0% |
| 7.70 | | 0% |
| 7.75 | | 0% |
| 7.80 | | 0% |
| 7.85 | | 0% |
| 7.90 | | 0% |
| 7.95 | | 0% |
| 8.00 | | 0% |
| 8.05 | | 0% |
| 8.10 | | 0% |
| 8.15 | | 0% |
| 8.20 | | 0% |
| 8.25 | | 0% |
| 8.30 | | 0% |
| 8.35 | | 0% |
| 8.40 | | 0% |
| 8.45 | | 0% |
| 8.50 | | 0% |
| 8.55 | | 0% |
| 8.60 | | 0% |
| 8.65 | | 0% |
| 8.70 | | 0% |
| 8.75 | | 0% |
| 8.80 | | 0% |
| 8.85 | | 0% |
| 8.90 | | 0% |
| 8.95 | | 0% |
| 9.00 | | 0% |
| 9.25 | | 0% |
| 9.50 | | 0% |
| 9.75 | | 0% |
| 10.00 | | 0% |
| 10.25 | | 0% |
| 10.50 | | 0% |
| 10.75 | | 0% |
| 11.00 | | 0% |
| 11.25 | | 0% |
| 11.50 | | 0% |
| 11.75 | | 0% |
| 12.00 | | 0% |

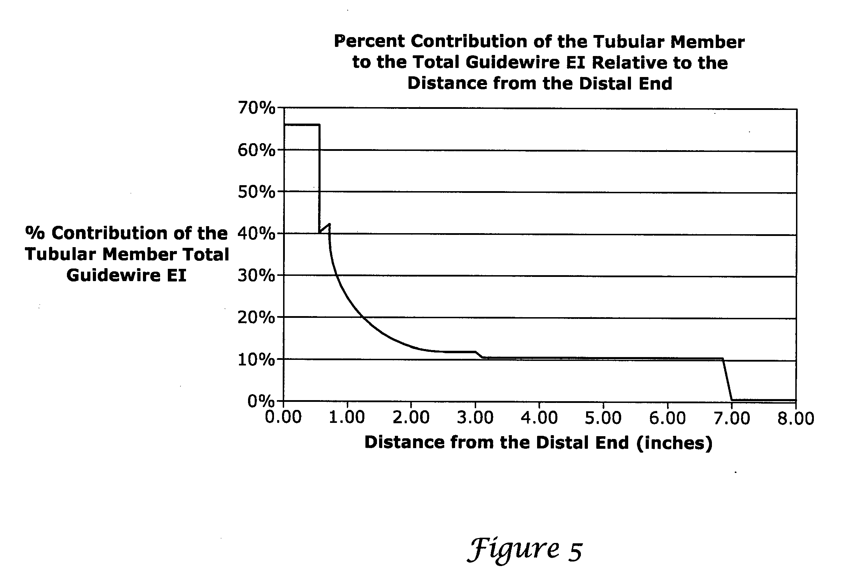

The results were plotted in Figure 5.

It should be understood that this disclosure is, in many respects, only illustrative. Changes may be made in details, particularly in matters of shape, size, and arrangement of steps without exceeding the scope of the invention. The invention's scope is, of course, defined in the language in which the appended claims are expressed.

## Claims

1. A medical device (10), comprising:
an elongate core member (18) having a proximal section (22) and a distal section (24);
a tubular member (20) disposed about the distal section (24) of the core member (18), the tubular member (20) having a plurality of slots (36) formed therein; and
a tip member (28);
wherein the distal section (24) of the core member (18) and the tubular member (20) define a distal tip (26) for the medical device (10), the distal tip (26) having a proximal end (32) and a distal end (34) and being free of a shaping ribbon;
the medical device (10) being **characterized by**:
the elongate core member (18) including a super elastic nickel-titanium alloy;
the tubular member (20) including a nickel-chromium-molybdenum alloy;
wherein the distal section (24) of the core member (18) is coupled to the tip member (28);
wherein adjacent to the proximal end (32) of the distal tip (26), the distal tip (26) has super elastic characteristics dominated by the shapability characteristics of the super elastic core member (18);
wherein adjacent to the distal end (34) of the distal tip (26), the distal tip (26) is shapeable, and
wherein between the proximal end (32) of the distal tip (26) and the distal end (34) of the distal tip (26), the core member (18) tapers so that the distal tip (26) transitions from having the super elastic characteristics of the core member (18) to being shapeable.

2. The medical device of claim 1, wherein the distal tip (26) is free of a coil.

3. The medical device of claim 1 or 2, wherein the tubular member (20) has a longitudinal axis, wherein the slots (36) formed in the tubular member (20) are arranged in groups of slots that are disposed at the same longitudinal position along the longitudinal axis of the tubular member (20), and wherein the groups each include two slots.

4. The medical device of any one of claims 1 to 3, wherein the tubular member (20) has a longitudinal axis, wherein the slots (36) formed in the tubular member (20) are arranged in groups of slots that are disposed at the same longitudinal position along the longitudinal axis of the tubular member (20), and wherein the groups each include three slots.

## Patentansprüche

1. Medizinische Vorrichtung (10), mit:
einem länglichen Kernelement (18) mit einem proximalen Abschnitt (22) und einem distalen Abschnitt (24);
einem röhrenförmigen Element (20), das um den distalen Abschnitt (24) des Kernelements (18) angeordnet ist, wobei das röhrenförmige Element (20) mehrere darin ausgebildete Schlitze (36) aufweist; und
einem Spitzenelement (28);
wobei der distale Abschnitt (24) des Kernelements (18) und das röhrenförmige Element (20) eine distale Spitze (26) für die medizinische Vorrichtung (10) definieren, wobei die distale Spitze (26) ein proximales Ende (32) und ein distales Ende (34) aufweist und frei von einem Formungsband ist;
wobei die medizinische Vorrichtung (10) **dadurch gekennzeichnet ist, dass**:
das längliche Kernelement (18) eine superelastische Nickel-Titan-Legierung aufweist;
das röhrenförmige Element (20) eine Nickel-Chrom-Molybdän-Legierung aufweist;
wobei der distale Abschnitt (24) des Kernelements (18) mit dem Spitzenelement (28) gekoppelt ist;
wobei benachbart zum proximalen Ende (32) der distalen Spitze (26) die distale Spitze (26) superelastische Eigenschaften aufweist, die durch die Formbarkeitseigenschaften des superelastischen Kernelements (18) beherrscht werden;
wobei benachbart zum distalen Ende (34) der distalen Spitze (26) die distale Spitze (26) formbar ist, und
wobei sich zwischen dem proximalen Ende (32) der distalen Spitze (26) und dem distalen Ende (34) der distalen Spitze (26) das Kernelement (18) verjüngt, so dass die distale Spitze (26) davon, dass sie die superelastischen Eigenschaften des Kernelements (18) aufweist, dazu übergeht, formbar zu sein.

2. Medizinische Vorrichtung nach Anspruch 1, wobei die distale Spitze (26) frei von einer Spirale ist.

3. Medizinische Vorrichtung nach Anspruch 1 oder 2, wobei das röhrenförmige Element (20) eine Längsachse aufweist, wobei die im röhrenförmigen Element (20) ausgebildeten Schlitze (36) in Gruppen von Schlitzen angeordnet sind, die an derselben longitudinalen Position längs der Längsachse des röhrenförmigen Elements (20) angeordnet sind, und wobei die Gruppen jeweils zwei Schlitze umfassen.

4. Medizinische Vorrichtung nach einem der Ansprüche 1 bis 3, wobei das röhrenförmige Element (20) eine Längsachse aufweist, wobei die im röhrenförmigen Element (20) ausgebildeten Schlitze (36) in Gruppen von Schlitzen angeordnet sind, die an derselben longitudinalen Position längs der Längsachse des röhrenförmigen Elements (20) angeordnet sind, und wobei die Gruppen jeweils drei Schlitze umfassen.

## Revendications

1. Dispositif médical (10), comprenant :
un élément central longitudinal (18) présentant une section proximale (22) et une section distale (24) ;
un élément tubulaire (20) disposé autour de la section distale (24) de l'élément central (18), l'élément tubulaire (20) présentant une pluralité de fentes (36) ménagés dans celui-ci ; et
un élément d'embout (28) ;
où la section distale (24) de l'élément central (18) et l'élément tubulaire (20) définissent un embout distal (26) pour le dispositif médical (10), ledit embout distal (26) présentant une extrémité proximale (32) et une extrémité distale (34) et étant exempt de ruban de formage ;
ledit dispositif médical (10) étant **caractérisé en ce que** :
l'élément central longitudinal (18) contient un alliage super-élastique nickel-titane ;
l'élément tubulaire (20) contient un alliage nickel-chrome-molybdène ;
la section distale (24) de l'élément central (18) étant reliée à l'élément d'embout (28) ;
l'embout distal (26) présentant des caractéristiques super-élastiques dominées par les caractéristiques de formabilité de l'élément central (18) super-élastique dans la partie adjacente à l'extrémité proximale (32) de l'embout distal (26) ;
l'embout distal (26) étant formable dans la partie adjacente à l'extrémité distale (34) de l'embout distal (26), et
l'élément central (18) étant aminci entre l'extrémité proximale (32) de l'embout distal (26) et l'extrémité distale (34) de l'embout distal (26), de telle manière que l'embout distal (26) passe des caractéristiques super-élastiques de l'élément central (18) à la formabilité.

2. Dispositif médical selon la revendication 1, où l'embout distal (26) est exempt d'enroulement.

3. Dispositif médical selon la revendication 1 ou la revendication 2, où l'élément tubulaire (20) présente un axe longitudinal, où les fentes (36) ménagées dans l'élément tubulaire (20) sont agencées en groupes de fentes disposées au même emplacement longitudinal le long de l'axe longitudinal de l'élément tubulaire (20), et où chaque groupe comprend deux fentes.

4. Dispositif médical selon l'une des revendications 1 à 3, où l'élément tubulaire (20) présente un axe longitudinal, où les fentes (36) ménagées dans l'élément tubulaire (20) sont agencées en groupes de fentes disposées au même emplacement longitudinal le long de l'axe longitudinal de l'élément tubulaire (20), et où chaque groupe comprend trois fentes.
